Europäisches Patentamt

European Patent Office

Office européen des brevets

(11) Publication number: **0 337 288**
**A1**

## (12) EUROPEAN PATENT APPLICATION

(21) Application number: 89106029.5

(22) Date of filing: 06.04.89

(51) Int. Cl.⁴: **A61B 17/58**

(30) Priority: 11.04.88 IT 478588 U

(43) Date of publication of application:
**18.10.89 Bulletin 89/42**

(84) Designated Contracting States:
**AT BE CH DE ES FR GB GR LI LU NL SE**

(71) Applicant: **Armillei, Egisto**
**Via Vocabolo Piedimonte 1/B**
**I-05100 Terni(IT)**

(72) Inventor: **Armillei, Egisto**
**Via Vocabolo Piedimonte 1/B**
**I-05100 Terni(IT)**

(74) Representative: **Modiano, Guido et al**
**MODIANO, JOSIF, PISANTY & STAUB**
**Modiano & Associati Via Meravigli, 16**
**I-20123 Milan(IT)**

(54) Device for reducing and fixing fractures of the hip joint.

㉗ The device for reducing and fixing fractures of the hip joint comprises a plate (3) adapted to be fixed with screws (5) to the body of a femur (2) and having a hollow cylinder (6) for guiding a femur head fixing screw (7). The cylinder (6) has an internal seat for accommodating a locking nut (8), screwed onto a threaded portion (7a) of the fixing screw (7) and defining an abutment cooperating with a spring (11) for forcing the surfaces of the fracture together.

Fig.3

EP 0 337 288 A1

## DEVICE FOR REDUCING AND FIXING FRACTURES OF THE HIP JOINT

The present invention relates to a device for reducing and fixing fractures of the hip joint, and in particular for reducing and fixing pre-trochanteric and sub-trochanteric fractures of the femur.

As known, the cure of fractures of the femur at the hip joint, including those of the trochanteric region, is still a difficult problem to solve. The healing process of such fractures is in fact linked to the extent of the vascular alteration, i.e. to the circulation deficit which arises with the mechanism of fracture. It is therefore necessary first of all, to achieve the anatomical reconstruction of the fracture, since a perfect alignment facilitates the healing process. It is thus necessary to carry out a valid osteosynthesis, performed with means which ensure the fixing of the stumps.

From a biomechanical point of view, the proximal end of the femur somewhat resembles a rigid column surmounted by a transverse bar arranged eccentrically with a single lever arm, i.e. the hip joint of the femur. If this lever arm is loaded, pressure, traction and flexure forces are exerted; said forces are opposed by the action of the counterweight forces. In the case of a fracture of the hip joint of the femur, this functional balance is lost, with more or less severe pathological aspects which must be eliminated by means of an intervention which allows the anatomical reconstruction of the injured part; the functional balance thus restored must be maintained with a valid osteosynthesis.

Various synthesis means have been used for this purpose so far; in the course of time, however, such means generally cause the diastasis of the fracture stumps which consequently fail to consolidate.

The aim of the present invention is to solve the above described problem by providing a device for reducing and fixing fractures of the hip joint, which ensures the correct coupling, in the course of time, of the surfaces of the fracture and which maintains a constant pressure on said surfaces.

Within the scope of this aim, a further object of the present invention is to provide a device for reducing and fixing fractures of the hip joint which is simple in concept, safe and reliable in use, and causes the minimum possible damage to the involved bone and muscle tissues.

This aim and this object are both achieved, according to the invention, by a device for reducing and fixing fractures of the hip joint comprising at least one plate, adapted to be applied with screw means to the body of the femur and having at least one hollow cylinder for guiding a femur head fixing screw, characterized in that said hollow guiding cylinder is internally provided with at least one seat for accommodating at least one locking nut, screwed in an adjustable position on a threaded portion of said fixing screw and defining abutment means cooperating with elastic biasing means, so as to provide an elastic thrust for forcing the fracture surfaces together.

The details of the invention will become apparent from the detailed description of a preferred embodiment of the device for reducing and fixing fractures of the hip joint, illustrated only by way of non-limitative example in the accompanying drawings, wherein:

figure 1 is a perspective view of the device for reducing and fixing fractures of the hip joint;

figure 2 is a front elevational view thereof;

figure 3 is a longitudinal sectional view of the device according to the invention, applied to the femur for reducing and fixing a fracture of the hip joint; and

figure 4 is a transverse sectional view of the plate of the device according to the invention, taken along the line IV-IV of figure 2.

With particular reference to the above described figures, the reference numeral 1 generally indicates the device for reducing and fixing fractures of the hip joint of the femur 2. The device 1 is substantially constituted by a plate 3 which has a plurality of holes 4 for the screws 5 for fixing it to the body of the femur. The profile of the plate 3 is complementary to that of the femur 2, so as to provide contact on a large surface without producing individual pressure points which may cause concentrations of stresses and tensions in the bone; in particular, the plate has an upper portion 3a which is slightly inclined so as to rest on the major trochanter 2a of the femur.

A hollow guide cylinder 6 is associated with the plate 3 on the longitudinal median plane thereof, and is adapted to guide a fixing screw 7 for the head 2b of the femur. The cylinder 6 is inclined upwards so as to be as parallel as possible to the axis of the hip joint of the femur. Since the hip joint of the femur may have different lateral inclinations with respect to the longitudinal axis of the femur, the device is conveniently manufactured with different angles of the cylinder 6; in particular, the axis of the cylinder 6 is inclined, with respect to the axis of the femur body, at an angle comprised between 125 and 135 degrees and is preferably provided in different versions with fixed angles equal to 125, 130 and 135 degrees, to suit different hip joint configurations.

The fixing screw 7 has, at one end, a threaded portion 7a on which a locking screw 8 is intended to screw and, at the opposite end, a thread 9 with a conical profile to provide grip inside the head of the femur. More in detail, e.g., the thread 9 may extend for a length of 25 mm, with a thread height comprised between 0 and 2.25 mm, a thickness of 1.4 mm at the lower base of the threads and a pitch of 3.15 mm.

The cylinder 6 is internally provided with a coaxial cavity having a widened diameter which is delimited by a shoulder 10 and defines a seat for the accommodation of the locking nut 8. An end of a helical spring 11 is adapted to abut on the shoulder 10; at its other end, said spring acts in compression on the nut 8, with the interposition of a washer-like member, constituted by e.g., a polyethylene washer 12. The nut 8 advantageously has cross-shaped grooves 8a adapted to allow its screwing on the fixing screw 7; the screw 7 expediently ends, in turn, on the side of the threaded portion 7a, with a polygonal or square head 7b adapted to allow it to be screwed onto the head of the femur by means of a suitable tool.

In practice, an accommodation for the cylinder 6 is provided in the femur; the fixing screw 7, which is inserted through the cavity of said cylinder, is screwed inside the head 2b of the hip joint of the femur by means of the thread 9. The spring 11 is then introduced axially onto the screw 7 and inserted inside the cavity of the cylinder 6, and is compressed by the locking nut 8 screwed on the threaded portion 7a of said screw 7.

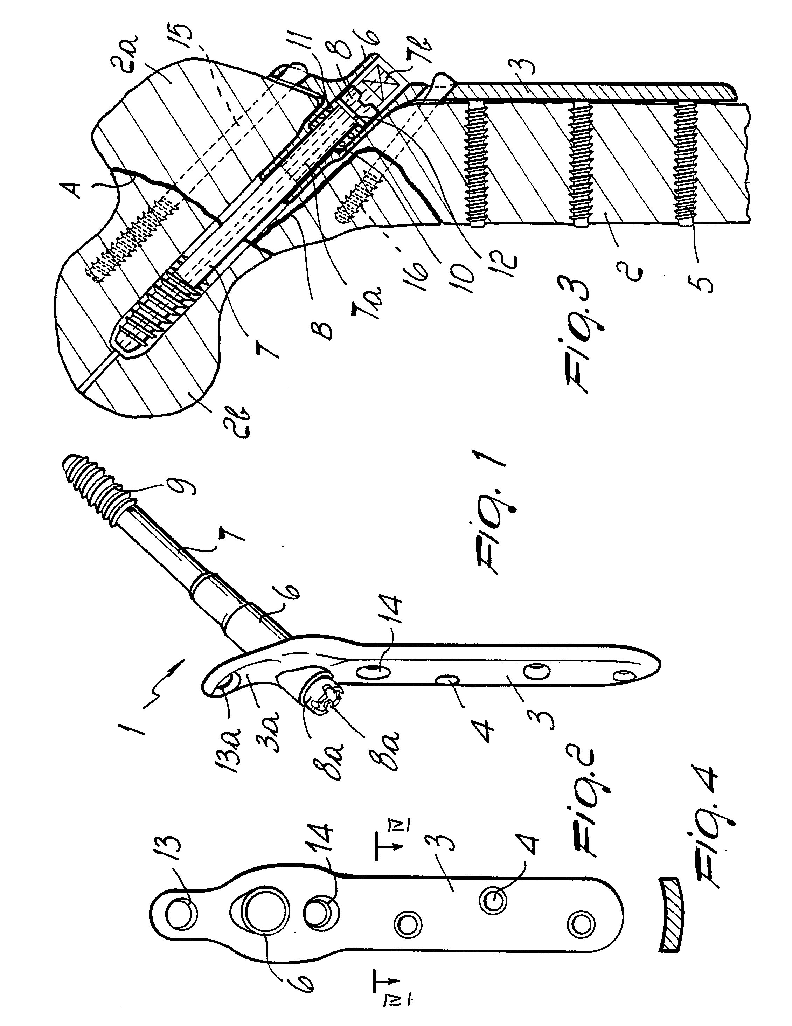

Besides achieving a safe locking of the fixing screw 7, the nut 8 therefore also permits, upon being tightened, an elastic thrust to be generated for causing mutual approach of the surfaces of the fracture, indicated by the line A in figure 3. Said thrust is conveniently adjustable by screwing the locking nut 8, which can move axially in the seat defined inside the guide cylinder 6. It should be noted that the nut 8 furthermore constitutes an alignment element for the screw 7.

In order to avoid any possible rotation of the head 2b of the femur about the fixing screw 7, a pair of holes 13 and 14 is provided in the plate 3 immediately above and below the cylinder 6; the axis of said holes is substantially parallel to that of said cylinder. Respective screws 15 and 16 are inserted in the holes 13 and 14 and engage in the femur with an appropriately variable angle. The lower screw 16 furthermore allows to fix a possible third fragment which may be present, as indicated by the fracture line B in figure 3.

To summarize, the described device allows the simultaneous achievement of a series of important advantages, and in particular to ensure and maintain the surfaces of the fracture mutually forced together or coapted in the course of time by virtue of the action of the spring 11, to prevent the rotation of the femur head fixing screw 7, to ensure a firm grip by virtue of the particular configuration of the conical thread 9 of said screw, and to minimize damage and irritations to the muscle tissue due to the complete absence of protrusions from the plate 3. It should be noted, in fact, that in an operating configuration, the end of the screw 7 and the nut 8 are completely embedded within the cylinder 6.

Finally, the simplicity of use of the device is evident; said device effectively solves the problem of fixing the fracture stably in the course of time, allowing at the same time a good mobility of the patient.

In the practical embodiment of the invention, the materials employed, as well as the shapes and dimensions, may be any according to the requirements.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. Device for reducing and fixing fractures of hip joint comprising at least one plate (3) adapted to be applied by screw means (5) to the body of the femur (2) and having at least one hollow cylinder (6) for guiding a femur head (2b) fixing screw (7), characterized in that said hollow guiding cylinder (6) is internally provided with at least one seat for accommodating at least one locking nut (8), screwed in an adjustable position on a threaded portion (7a) of said fixing screw and defining abutment means cooperating with elastic biasing means (11) so as to provide an elastic thrust for forcing the surfaces of the fracture together.

2. Device according to claim 1, characterized in that said guiding cylinder (6) is inclined, with respect to the axis of the body of the femur, by an angle comprised between 125 and 135 degrees.

3. Device according to claim 1, characterized in that said seat for the accommodation of the locking nut (8) is defined by a cavity having a wider diameter than the guiding cylinder (6), said cavity being delimited by a shoulder (10) on which an end of said spring (11) is adapted to abut, said spring acting, at its other end, by compressing on said nut (8) by virtue of the interposition of a polyethylene washer (12).

Fig. 2

Fig. 4

Fig. 1

Fig. 3

EP 0 337 288 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | US-A-2 801 631 (J. CHARNLEY) <br> * Whole document * | 1,2 | A 61 B 17/58 |
| Y | | 3 | |
| Y | US-A-3 596 656 (B.B. KAUTE) <br> * Column 2, lines 63-70; figures * | 3 | |
| A | DE-A-1 055 750 (G.-B.L. GRATH) | | |
| A | BE-A- 524 818 (E. POHL) | | |

TECHNICAL FIELDS SEARCHED (Int. Cl.4)

A 61 B

The present search report has been drawn up for all claims

| Place of search | Date of completion of the search | Examiner |
|---|---|---|
| THE HAGUE | 01-07-1989 | STEENBAKKER J. |